Europäisches Patentamt

(19) European Patent Office

Office européen des brevets

(11) Publication number: **0 253 684 B1**

# EUROPEAN PATENT SPECIFICATION

(45) Date of publication of patent specification: **10.04.91**  (51) Int. Cl.⁵: **A61K 9/24**, A61K 9/56, A61K 9/16

(21) Application number: **87306385.3**

(22) Date of filing: **17.07.87**

(54) Substained-release formulation and production thereof.

(30) Priority: **17.07.86 JP 169255/86**

(43) Date of publication of application:
**20.01.88 Bulletin 88/03**

(45) Publication of the grant of the patent:
**10.04.91 Bulletin 91/15**

(84) Designated Contracting States:
**AT BE CH DE ES FR GB GR IT LI LU NL SE**

(56) References cited:
**EP-A- 0 164 959
DE-A- 2 010 416
DE-B- 1 001 456
DE-C- 835 500
US-A- 4 572 833**

(73) Proprietor: **SHIONOGI SEIYAKU KABUSHIKI KAISHA trading under the name of SHIONOGI & CO. LTD.
12, 3-chome, Dosho-machi Higashi-ku Osaka(JP)**

(72) Inventor: **Sakamoto, Teruo
11-10, Nagisasakaemachi
Hirakata-shi Osaka(JP)**
Inventor: **Takeda, Toyohiko
44-8, Sumiyoshidai Higashinada-ku
Kobe-shi Hyogo(JP)**
Inventor: **Suzuki, Yusuke
5-15-5, Fuchu-cho
Izume-shi Osaka(JP)**
Inventor: **Noda, Kinzaburo
3-418, Ikejiri
Itamai-shi Hyogo(JP)**
Inventor: **Fujii, Toshiro
1-1-1, Mukoyama
Takarazuka-shi Hyogo(JP)**

(74) Representative: **Hardisty, David Robert et al
BOULT, WADE & TENNANT 27 Furnival Street
London EC4A IPQ(GB)**

## Description

The present invention provides sustained-release formulations of a pharmacologically active ingredient or ingredients, especially of those which have been hardly formulated because of their high solubility in water (hereinafter sometimes referred to as water-soluble active ingredients), and methods for preparing sustained-release formulations of them are also provided.

A number of reports have been publisher on the techniques for effectively controlling dissolution and absorption of water-soluble pharmacologically active ingredients, especialy of active ingredients which are very soluble in gastric or intestinal juice.

These techniques for sustainment of drugs may be classified generally into two types. One is achieved by forming a matrix and the other is by film-coating.

Thus, for example, sustained-release has been accomplished by granulating an active ingredient together with a hydrophobic substance to form matrices as disclosed in Japanese Patent Publication No. 60-56122. However, there is no disclosure on release-control over four hours in the publication above.

In JPN Patent Application No. 61-1614, sustained-release has been accomplished by making triple layers with three different types of pH-dependent film-coating materials. In the above process, however, troublesome coating steps are required and moreover a large amount of coating materials must be employed for a limited amount of the active component in order to achieve appropriate sustained-release. Moreover, the use of such a large amount of coating materials unfavourably decreases bio-availability of the active ingredients.

DE-B-1001456 discloses a sustained release preparation having an inert core, an active ingredient located in a first powder coating and a retarding coating which is sprayed on in diluted form.

The present invention provides sustained-release formulations which can release the active ingredient or ingredients in a zero-order release pattern over a long period of time, comprising:

a. an inert core;
b. a powder-coating layer containing a water-soluble active ingredient or ingredients,
c. a powder-coating layer of practically water-repellent material, and
d. film-coating layer composed of practically pH-independent and water-soluble film-coating material.

In methods for sustained-release using the formation of matrices of film-coating layers, the pattern of drug-release is very similar to a first-order-like one and controllable drug-release duration is limited to at most a few hours. In order to give a zero-order release pattern to the formulation, it is necessary to use a large amount of material for forming matrices or film-coating layers, but this operation is accompanied with some defects such as a decrease of bio-availability of active ingredients.

The present invention provides sustained-release formulations or means by which active ingredients are released in a zero-order release manner, i.e. at a constant rate, over a period of 10 hours of longer. The present invention is very useful in preparing sustained-release formulations of active ingredients such as those which are to be absorbed over a large area of the digestive organs, and which act quickly but shortly. According to the present invention, formulations having desirable drug-release patterns, for example, rapidly acting but long-lasting formulations and the like can easily be prepared by means of prior art techniques such as combinations of slow-release preparations with rapid-release ones.

The present invention is based upon the finding that zero-order release of pharmacolgically active ingredients over a long period of time is realized by providing a powder-coating layer containing the pharmacologically active ingredient on a core, and further making thereon a powder-coating layer of a practically water-relellent material, and then a film-coating layer of a practically pH-independent and water-insoluble material

The sustained-release formulations of the present invention may be prepared in the following manner. The core materials may be coated with powder which contains a water-soluble active ingredient or ingredients in the presence of a water-insoluble or very slightly soluble binding agent to give bare granules, and then said bare granules are coated with a water-repellent powder so that the weight increases by 10% to 80%, then dried, further coated with a practically pH-independent and water-insoluble film-coating material, if desired together with an enteric-coating material, a lubricant, a plasticizer, or the like, until the weight increases by 20 to 60%, more preferably about 35 to 45%, and finally dried.

In this process the drying steps should be operated at a lower temperature than the melting point of any water-repellent materials. If it is done at a higher temperature such water-repellent materials might be fused resulting in interference with desirable release of the active ingredient(s).

The mechanism of zero-order release exhibited by the thus-prepared formulations of the present invention may be explained as follows. When the formulation is orally administered and reaches the stomach the moisture of the gastric juice permeates into the film-coating layer and then into the double

2

power-coating layers gradually to dissolve out the active ingredient from the surface of the inner powder-coating layer. The dissolved active ingredient is dispersed in the outer powder-coating layer and slowly released through the film-coating layer. At that time, the concentration of the active ingredient is kept constant in the outer powder-coating layer and, as a result, zero-order release is maintained over a long period of time.

Any material can be employed as the core material of this invention as long as it is pharmacologically inert and exhibits no interaction with the water-soluble pharmacologically active ingredients used. Saccarides or sugar alcohols such as sucrose, lactose, mannitol, xylitol and the like; various celluloses; various starches; and the like are exemplified. They may be employed in the form of crystals or singly or as a mixture in the form of granules or beads.

The term "the powder containing a water-soluble pharmacologically active ingredient" also indicates the active ingredient per se and, more preferably, a mixture of powder of the active ingredient with suitable excipients may be employed in order to improve operability in the coating process. The active ingredients are released at a high speed if a hydrophilic material is used as the excipient and, on the contrary, the release rate can be reduced if a hydrophobic one is used. Furthermore, sustained-release formulations of slightly soluble active ingredients can be made by using hydrophilic materials.

Water-repellent materials include hardened glycerol fatty acid esters such as hardened castor oil, hardened beef tallow or the like; higher fatty acids such as stearic acid or the like; higher fatty acid metal salts such as magnesium stearate, calcium stearate, or the like; higher alcohols such as stearyl alcohol, centanol, or the like; and waxes such as carnauba wax, beeswax, or the like. They may be employed singly or in a mixture, or two or more species of them may be applied to form a multilayer coating.

Although the amounts of those materials to be used should not be limited imprudently (because they vary with the sort of materials used, the density or size of the uncoated bare preparation, as well as the solubility of the active ingredient to be used), they may be used generally at an amount of about 10 to 80% by weight to the bare granules. The use of them at higher amounts reduces availability (capability of release) of the active ingredient, and at lower amounts retardation or release is not sufficient

Suitable pH-independent and water-insoluble film-coating materials include ethylcellulose, copolymers of ethyl methacrylate and trimethylammoniumethyl chloride methacrylate (Eudragit® RS), shellac, highly polymerized polyvinyl alcohol, water-insoluble polyvinyl pyrrolidone, polyvinyl chloride, cellulose acetate, polyurethane, tetrafluoroethylene, polystyrene, polypropylene, lactic acid polymers, hydroxyethyl methacrylate, glycolic acid polymers, polyethylene terephthalate, polyethylene, polyamides, polyacrylonitrile, polycarboxylic acids, cyanoacrylic acid polymers, and the like. Ethylcellulose and copolymers of ethyl methacrylate and trimethylammoniumethyl chloride methacrylate may preferably be employed.

The film-coating materials may be employed together with suitable additives, if necessary. "Additive" includes enteric coating materials, water-soluble coating materials, lubricants, plasticizers, or the like. Enteric coating materials include hydroxypropylmethylcellulose phthalate (HPMCP)< hydroxypropylmethylcellulose acetate succinate (HPMC-AS), copolymers of methacrylic acid and methyl methacrylate, cellulose acetate phthalate (CAP), and the lime. Water-soluble coating materials include methylcellulose (MC), hydroxypropylcellulose (HPC), polyvinyl pyrrolidone (PVP), polyvinyl alcohol (PVA), gelatin, hydroxypropylmethylcellulose (HPMC), polycarboxylic acid (Carbopol®), starches, sodium alginate, and the like. Lubricants include talc, stearic acid, magnesium stearate, and the like. Plasticizers include triacetin and the like.

The amount of film-coating materials to be used varies with the sort of the amount of additives. Generally, the materials may be employed at an amount of 20 to 60%, preferably 35-45% compared to the bare granules. Availability of the active ingredient decreased rapidly when the coating material is used over the upper limit, and retardation also decreased rapidly at below the said lower limit.

In the sustained-release formulations of the present invention, the release-rate of the active ingredient is exactly controllable depending upon the powder-coating layer of practically water-repellent material and, as a result, this minimizes the amount of film-coating material necessary. Therefore, almost 100% of the active ingredient can be released from the formulation at a constant rate, even if it is so designed as to act over a 10-hour period.

Because the sustained-release formulations of the present invention are pH-independent, they are expected to have the same release properties in aged persons or persons with hypochlorhydria or anacidity as in healthy persons.

Many sustained-release formulations which consist essentially of hardened oils, triglycerol, and the like have been described in the prior art, but they have disadvantages such as sensibility to digestive enzymes including lipases, esterases, and the like, or bile acids. On the other hand, the formulations of the present invention are not influenced by such things.

3

The present invention is explained in more detail by the following Examples and Experiments, which are not intended to limit the scope of the present invention.

In the accompanying drawings:-

Figure 1 shows the dissolution rate of PPA on the formulations of the present invention prepared as in Examples 8 to 12. The numerals 1 to 5 in the Figure indicate the formulations of Examples 8 to 12, respectively.

Figure 2 shows the dissolution rate of d-chlorpheniramine maleate on the formulations of the present invention prepared as in Examples 13 to 16. The numerals 1 to 4 in the Figure indicate the formulations of Examples 13 to 16, respectively.

Figure 3 shows the dissolution rate of belladonna total alkaloids on the formulation of the present invention prepared as in Example 17.

Figure 4 shows the dissolution rate of PPA on the formulation of the prior art prepared as in Experiment 4. The numerals 1 to 3 in the Figure indicate the Reference Formulations No. 1 to 3, respectively.

Figure 5 shows the dissolution rate of PPA in the three kinds of test fluids (a, b, and c) listed in Experiment 5 on the present formulation of Example 8 and on the Reference Formulation No. 2 prepared as in Experiment 4. In the figure, the numerals 1 and 2 indicate Reference Formulation No. 2 and the formulation of the present invention prepared as in Example 8, respectively.

Example

(General Procedures for Sustained-release Formulations)

In the presence of additives, cores are coated with powder containing an active ingredient or ingredients to give bare preparations, each of which has a drug layer. The bare preparations are, in the presence of additives, powder-coated with water-repellent materials and then dried at 60°C for an hour with forced aeration. Thus prepared granules are coated with film-coating materials by a pan-coating method and then dried at 80°C for 20 minutes with forced aeration to give spherical sustained-granules.

Reference Example 1

(Preparation of Cores)

Mixed powder of 2500g D-mannitol, 200g crystalline cellulose, and 650g corn starch is triturated through a 80-mesh screen, and granulated, in the presence of 2% aqueous solution of methylcellulose as a binder, by a wetting method with a Supermixer (made by Kawata Seksakusho) to give four different sizes of granules, i.e. mean particle sizes about 230μm, about 320μm, about 500μm, and about 710μm.

Reference Example 2

(Preparation of Rapid-release Formulation)

200g of the granules about 320μm in mean particle size which were prepared according to Reference Example 1 were coated with a mixed powder of 250g phehylpropanolamine chloride, 1g belladonna total alkaloids and 300g D-mannitol, which was triturated in advance through a 80-mesh screen by using a Supermixer, while 500g of a 2% aqueous solution of methylcellulose 25mPas as a binder was sprayed thereon at a rate of 12g per minute. The granules were dried at 60°C for a hour with forced aeration to give spherical rapid-release graules of about 480 m in mean particle size. The resulting granules contain 332mg of phenylpropanolamine chloride and 1.3mg of belladonna total alkaloids per gram.

This formulation is used as a long-acting formulation by combining with a sustained-release formulation prepared in the following Examples.

4

Reference Example 3

(Preparation of Rapid-release Formulation)

According to the method of Reference Example 2, a rapid-release formulation was prepared using 200g of granules of about 320μm in mean particle size. The granules contain 333mg of phenylpropanolamine chloride per gram and are about 480μm in mean particle size.

This formulation is used as a long acting formulation by combining with a sustained-release formulation prepared in the following Examples.

Reference Example 4

(Preparation of Long-acting Formulation)

Gelatin hard capsules (size No. 2) were filled with 174mg of sustained-release granules prepared in Example 18 and 45mg of rapid-release granules prepared in Reference Example 2 to give a long-acting formulation, which contains 70mg of phenylpropanolamine chloride and 0.27mg of belladonna total alkaloids per capsule.

Reference Example 5

(Preparation of Long-acting Formulation)

Gelatin hard capsules (size No. 2) were filled with 158mg of sustained-release granules prepared in Example 8 and 60mg of rapid-release granules prepared in Reference Example 2 to give a long-acting formulation, which contains 70mg of phenylpropanolamine chloride per capsule.

Reference Example 6

(Preparation of Long-acting Formulation)

Gelatin hard capsules (size No. 2) were filled with 174mg of sustained-release granules prepared in Example 8 and 45mg of rapid-release granules prepared in Reference Example 2 to give a long-acting formulation, which contains 70mg of phenylpropanolamine chloride per capsule.

According to the General Procedures for Sustained-release Formulations, the following formulations were prepared.

Example 1

(Preparation of Bare Granules)

Using 200g of granules (about 320μm in mean particle size) as cores which were prepared according to Reference Example 1, a mixed powder of 400g of phenylpropanolamine chloride with 70g of hardened castor oil which was screened through 80-mesh was powder-coated onto the core granules by using a Supermixer, while 500g of a 2% ethanol solution of ethylcellulose 100mPas as a binder was sprayed thereon at a rate of 16g per minute, to form a layer of the active ingredient on said granules.

Example 2

(Preparation of Bare Granules)

In the same manner as in Example 1, a mixed powder of 40g d-chlorpheniramine maleate, 250g D-mannitol, 80g hardened caster oil, and 30g corn starch was coated onto granules (200g) of about 320μm in mean particle size to give a layer of the active ingredient thereon.

Example 3

(Preparation of Bare Granules)

Bare granules were prepared in the same manner as in Example 2 except that granules (200g) of about 230μm in mean particle size were used.

Example 4

(Preparation of Bare Granules)

Bare granules were parpared in the same manner as in Example 2 except that granules (200g) of about 500μm in mean particle size were used.

Example 5

(Preparation of Bare Granules)

Bare granules were prepared in the same manner as in Example 2 except that granules (200g) of about 710μm in mean particle size were used.

Example 6

(Preparation of Bare Granules)

In the same manner as in Example 1, a mixed powder of 1.5g of belladonna total alkaloids, 400g sucrose, and 68.5g hardened castor oil was coated onto granules (200g of about 320μm in mean particle size to give a layer of the active ingredient thereon.

Example 7

(Preparation of Bare Granules)

In the same manner as in Example 1, a mixed powder of 400g phenylpropanolamine chloride, 1.5g of belladonna total alkaloids, and 68.5g hardened castor oil was coated onto granules (200g) of about 320μm in mean particle size to give a layer of the active ingredients thereon.

Example 8

Onto the whole amount of bare granules prepared as in Example 1 was coated a mixed powder of 240g hardened caster oil with 80g magnesium stearate by a Supermixer, while 250g of a 5% ethanol solution of ethylcellulose 100mPas as a binde was sprayed thereon at a rate of 8g per minutes. The granules were dried at 60° C for an hour with forced aeration and, as a result, they came to have a powder-coading layer thereon at about 46% by weight of the bare granules.

A solution of 174g ethylcellulose, 8.7g methacrylic acid and methyl methacrylate copolymer, and 90g talc in a mixture of ethanol (1250g) with dichloromethane (3150g) was coated onto the granules by a pan-coating method to give film-coated granules, which were dried at 80° C for 20 minutes with forced aeration. There were spherical sustained-release granules of about 500μm in mean particle size, having a film-coating layer in each particle at about 40% by weight of the bare granules. The resulting granules contain 316mg of phenylpropanolamine chloride per gram.

In the following Examples and Experiments, the amount of powder-coating or of film-coating is shown as weight % of the bare granules used therefor.

Examples 9 to 12

The whole amount of bare granules prepared as in Example 1 were used in each Example to give the following spherical granules of about 500μm in mean particle size, which have different amounts of powder-coating layers.

The prescription for the powder-coating of film-coating is the same as in Example 8.

TABLE 1

| Example Number | Amount of Powder-coating | Amount of Film-coating | Amount of PPA in each gram |
|---|---|---|---|
| 9 | 24% | 40% | 358mg |
| 10 | 35% | 40% | 336mg |
| 11 | 55% | 40% | 301mg |
| 12 | 65% | 40% | 287mg |

**Note: PPA stands for phenylpropanolamine chloride.**

Example 13

Onto the whole amount of bare granules prepared as in Example 2 was coated a mixed powder of 500g of hardened castor oil with 100g of hardened beef tallow by a Supermixer, while 125g of a 10% ethanol solution of ethylcellulose 10mPas (binder) was sprayed thereon at a rate of 8g per minute. The granules were dried at 60° C for an hour with forced aeration and, as a result, they came to have a powder-coating layer thereon at about 43% by weight of the bare granules.

A film-coating dispersion of 174g of ethylcellulose, 20g hydroxypropylmethylcellulose phthalate-55 (HPMCP-55), and 100g stearic acid in a mixture of ethanol (1200g) with dichloromethane (3800g) was sprayed onto the resulting granules at a spraying-rate of 35g/min. The film-coated granules were dried at 80° C for 30 minutes with forced aeration to give spherical sustained-release granules of about 500μm in mean particle size, each particle of which has a film-coating layer at about 43% by weight of the bare granules. The granules contain 31.6mg of d-chlorpheniramine maleate per gram.

Example 14

7

Sustained-release granules were prepared in the same manner as in Example 13. A powder-coating layer (43%) and then a film-coating layer (43%) whose prescriptions are the same as in Example 13 were formed on all bare granules prepared as in Example 3 to give spherical sustained-release granules of 520 m in mean particle size. The granules contain 31.6mg of d-chlorpheniramine maleate per gram.

Example 15

Spherical sustained-release granules of 750$\mu$m in mean particle size were obtained in the same manner as in Example 14 except that bare granules as prepared in Example 4 were used. The granules contain 31.6mg of d-chlorpheniramine maleate per gram.

Example 16

Spherical sustained-release granules of 1500$\mu$m in mean particle size were obtained in the same manner as in Example 14 except that bare granules are prepared in Example 5 were used. The granules contain 31.6mg of d-chlorpheniramine maleate per gram.

Example 17

Spherical sustained-release granules of 500$\mu$m in mean particle size were obtained in the same manner as in Example 8 except that bare granules as prepared in Example 6 were used. The granules contain 1.19mg of belladonna total alkaloids per gram.

Example 18

Spherical sustained-release granules of 500$\mu$m in mean particle size were obtained in the same manner as in Example 8 except that bare granules as prepared in Example 7 were used. The granules contain 316 mg of phenylpropanolamine chloride and 1.19mg of belladonna total alkaloids per gram.

Experiment

Experiment 1

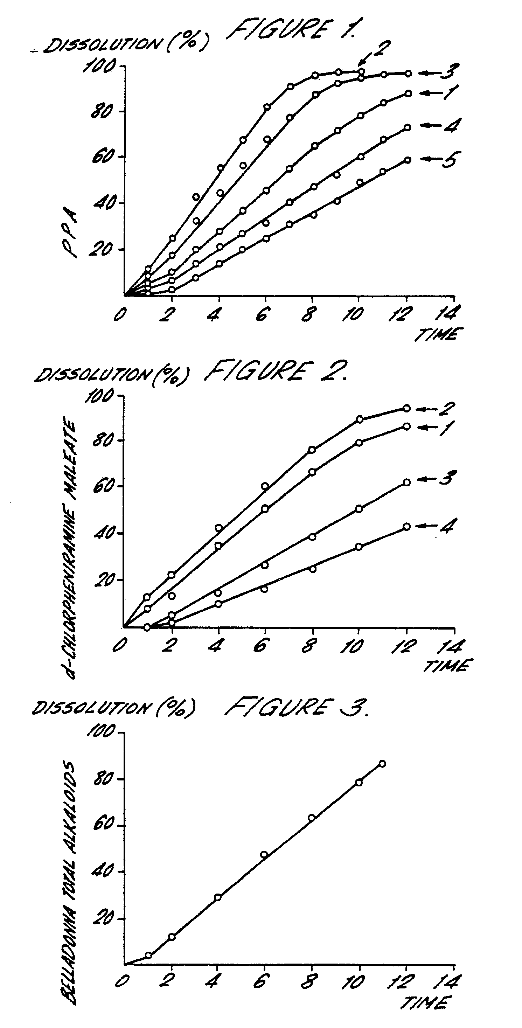

Dissolution tests (paddle method) were practiced on sustained-release granules prepared by Examples 8 to 12 in the second fluid (artificial intestinal juice) shown in JPN Pharmacopoeia X. The granules tested in each group contain 50mg of PPA. Every formulation showed a preferred zero-order release pattern (Figure 1).

Experiment 2

The same dissolution tests (paddle method) as in Experiment 1 were practiced on sustained-release granules prepared by Examples 13 to 16. Granules containing 5mg of d-chlorpheniramine maleate were employed for each test. The results shown that the larger the mean particle size the smaller the dissolution rate, and the granules showed the preferred zero-order release patterns independently of the size of the granules (Figure 2).

Experiment 3

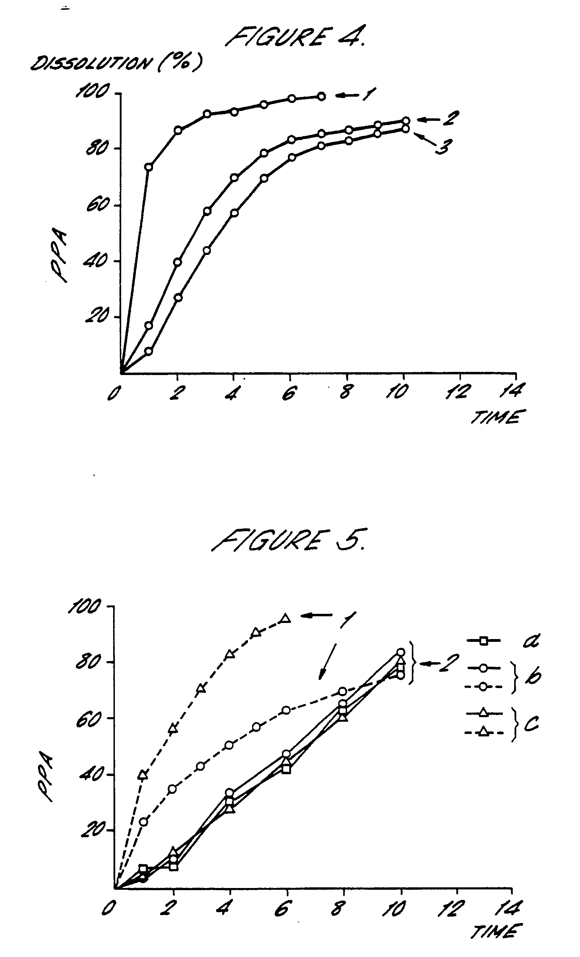

The same dissolution test (paddle method) as in Experiment 1 was practiced on sustained-release granules prepared by Example 17. Granules containing 0.3mg of belladonna total alkaloids were employed for each test. The granules showed the preferred zero-order release patterns even if the active ingredient therein was as little as 0.1 weight % compared to the granules (Figure 3).

Experiment 4

The formulations of the present invention were compared with those of the prior art prepared in the following manner (Figure 4).

(Reference Formulations 1 to 3 Production thereof)

The same film-coating solution as used in Example 8 was sprayed onto granules prepared as in Example 2 to give three kinds of sustained-release granules which have different amounts of coating-layers but are the same in size (mean particle size is 480µm). These formulations have no powder-coating layers.

## Table 2

| Reference Formulations (Prior Art) | Amount of Film Coating (%) | Amount of PPA in 1g of the Granules |
|---|---|---|
| 1 | 34.4 | 438mg |
| 2 | 37.3 | 428mg |
| 3 | 44.8 | 406mg |

From the results above, it can be understood that formulations of the prior art show the first-order release patterns and the dissolution rate cannot be controlled by changing the amount of the film-coating layer.

Experiment 5

In order to study the pH-dependency of the formulations of the present invention and the influence thereon of gastric enzymes, a dissolution test was practiced by using the following fluids (Figure 5).

Test Fluids

    a. the first fluid shown in JPN Pharmacopoeia X, and
    b. the second fluid shown in JPN Pharmacopoeia X, and
    c. mixture consisting of the following:
        the second fluid ---------- 900ml
        pancreatin ---------------- 0.7g
        lipase -------------------- 0.7g
        gall powder -------------- 2.0g

Tested Formulations

    The present invention ------- Example 8
    Reference ------------------- Reference Formulation No. 2

Granules containing 50mg of PPA were employed for the test.

The formulation of the present invention showed preferred zero-order release pattern independently of a change in pH value and of the presence or absence of gastric enzymes. On the other hand, the dissolution rate in the reference formulation was significantly influenced by gastric enzymes.

Preferable blood concentration-time curves were seen in a trial where the sustained-release formulation prepared in Reference Example 5 or 6 was administered to several healty adults. It was, therefore, confirmed that the formulations of the present invention would give clinically prererable drug-release patterns.

## Claims

1. A sustained-release formulation of a water-soluble active ingredient or ingredients comprising:
   a. an inert core;
   b. powder-coating layer containing the water-soluble active ingredient or ingredients;
   c. a powder-coating layer of a water-repellent material; and
   d. a film-coating layer composed of substantially pH-independent and water-insoluble film-coating material.

2. A sustained-release formulation as claimed in claim 1 wherein the water-repellent material, layer (c) and the water-insoluble film-coating material layer (d) are present in amounts of 10 to 80% and 20 to 60% by weight, based on the total weight of layers (a) and (b), respectively.

3. A process for the production of a sustained-release formulation of a water-soluble active ingredient or ingredients comprising: coating a material which contains the water-soluble active ingredient or ingredients in the presence of a water-insoluble or very slightly soluble binding agent onto a core material so as to make granules, coating the granules with a water-repellent material at an amount of 10 to 80% by weight based on the granules, and then coating a substantially pH-independent and water-insoluble film-coating material thereon at an amount of 20 to 60% by weight based on the granules.

4. A process as claimed in claim 3, wherein the material containing the active ingredient(s) and the water-repellent material are both powders.

5. A process as claimed in claim 3 or claim 4, wherein a drying step is employed after coating with water-repellent material and after coating with film-coating material.

6. A process as claimed in any one of claims 3 to 5, wherein the coating with film-coating material is effected together with an enteric-coating material and/or a lubricant and/or a plasticizer, or the like.

7. A process as claimed in any one of claims 3 to 6, wherein the coating with film-coating material is effected at an amount of 35 to 45% by weight based on the granules.

Claims for the following Contracting States : ES, GR

1. A process for the production of a sustained-release formulation of a water-soluble active ingredient or ingredients comprising:
   a. an inert core;
   b. a powder-coating layer containing the water-soluble active ingredient or ingredients;
   c. a powder-coating layer of a water-repellent material; and
   d. a film-coating layer composed of substantially pH-independent and water-insoluble film-coating material comprising: coating a material which contains the water soluble active ingredient or ingredients in the presence of a water-insoluble or very slightly soluble binding agent onto a core material so as to make granules, coating the granules with a water-repellent material, and then coating a substantially pH-independent and water-insoluble film-coating material thereon.

2. A process as claimed in claim 1 wherein the water-repellent material and the water-insoluble film-coating material are coated in amounts of from 10 to 80% and 20 to 60% by weight, based on the granules respectively.

3. A process as claimed in claim 1 or claim 2, wherein the material containing the active ingredient(s) and the water-repellent material are both powders.

4. A process as claimed in any one of claims 1 to 3, wherein a drying step is employed after coating with water-repellent material and after coating with film-coating material.

5. A process as claimed in any one of claims 1 to 4, wherein the coating with film-coating material is effected together with an enteric-coating material and/or a lubricant and/or a plasticizer, or the like.

6. A process as claimed in any one of claims 1 to 5, wherein the coating with film-coating material is effected at an amount of 35 to 45% by weight based on the granules.

7. A sustained-release formulation of a water-soluble active ingredient or ingredients comprising:
   a. an inert core;
   b. a powder-coating layer containing the water-soluble active ingredient or ingredients;
   c. a powder-coating layer of a water-repellent material; and
   d. a film-coating layer composed of substantially pH-independent and water-insoluble film-coating material.


**Revendications**

1. Composition, à libération prolongée, d'un ingrédient ou d'ingrédients actifs et solubles dans l'eau, comprenant :
   a. un noyau inerte,
   b. une couche d'enrobage pulvérulent, contenant l'ingrédient ou les ingrédients actifs et solubles dans l'eau,
   c. une couche d'enrobage pulvérulent, constituée d'une matière hydrofuge, et
   d. une couche d'enrobage pelliculaire, composée d'une matière d'enrobage à pH sensiblement neutre et insoluble dans l'eau.

2. Composition à libération prolongée selon la revendication 1, caractérisée en ce que la couche (c) de matière hydrofuge et la couche (d) de matière d'enrobage pelliculaire insoluble dans l'eau sont contenues selon des quantités respectives de 10 à 80% et de 20 à 60 % en poids par rapport au poids total des couches (a) et (b).

3. Procédé de préparation d'une composition, à libération prolongée, d'un ingrédient ou d'ingrédients actifs et solubles dans l'eau, caractérisé en ce qu'on applique une matière qui contient l'ingrédient ou les ingrédients actifs et solubles dans l'eau, en présence d'un liant insoluble ou très légèrement soluble dans l'eau, sur une matière de base de manière à produire des granules ; on recouvre les granules d'une matière hydrofuge à raison d'une quantité de 10 à 80 % en poids par rapport aux granules, puis on applique sur ladite matière hydrofuge une matière d'enrobage pelliculaire insoluble dans l'eau et à pH sensiblement neutre, selon une quantité de 20 à 60 % en poids par rapport aux granules.

4. Procédé selon la revendication 3, caractérisé en ce que la matière contenant le(s) ingrédient(s) actif(s) et la matière hydrofuge sont toutes les deux des poudres.

5. Procédé selon la revendication 3 ou 4, caractérisé en ce qu'on effectue une opération de séchage après l'enrobage au moyen de la matière hydrofuge et après l'enrobage au moyen de la matière d'enrobage pelliculaire.

6. Procédé selon l'une quelconque des revendications 3 à 5, caractérisé en ce que l'enrobage au moyen de la matière d'enrobage pelliculaire est réalisé conjointement avec une matière d'enrobage entérique et/ou un lubrifiant et/ou un plastifiant, ou analogues.

7. Procédé selon l'une quelconque des revendications 3 à 6, caractérisé en ce que l'enrobage au moyen de la matière d'enrobage pelliculaire est réalisé selon une quantité de 35 à 45 % en poids par rapport aux granules.

Revendications pour les Etats contractants suivants : ES, GR

1. Procédé de préparation d'une composition, à libération prolongée, d'un ingrédient ou d'ingrédients actifs et solubles dans l'eau, comprenant :
   a. un noyau inerte,
   b. une couche d'enrobage pulvérulent, contenant l'ingrédient ou les ingrédients actifs et solubles dans l'eau,
   c. une couche d'enrobage pulvérulent, constituée d'une matière hydrofuge, et
   d. une couche d'enrobage pelliculaire, composée d'une matière d'enrobage à pH sensiblement neutre et insoluble dans l'eau,
   caractérisé en ce qu'on applique une matière qui contient l'ingrédient ou les ingrédients actifs et solubles dans l'eau, en présence d'un liant insoluble ou très légèrement soluble dans l'eau, sur une matière de base de manière à produire des granules ; on recouvre les granules d'une matière hydrofuge, puis on applique sur ladite matière hydrofuge une matière d'enrobage pelliculaire insoluble dans l'eau et à pH sensiblement neutre.

2. Procédé selon la revendication 1, caractérisé en ce que la matière hydrofuge et la matière d'enrobage pelliculaire insoluble dans l'eau sont contenues selon des quantités respectives de 10 à 80% et de 20 à 60 % en poids par rapport aux granules.

3. Procédé selon la revendication 1 ou 2, caractérisé en ce la matière contenant le(s) ingrédient(s) actif(s) et la matière hydrofuge sont toutes les deux des poudres.

4. Procédé selon l'une quelconque des revendications 1 à 3, caractérisé en ce qu'on effectue une opération de séchage après l'enrobage au moyen de la matière hydrofuge et après l'enrobage au moyen de la matière d'enrobage pelliculaire.

5. Procédé selon l'une quelconque des revendications 1 à 4, caractérisé en ce que l'enrobage au moyen de la matière d'enrobage pelliculaire est réalisé conjointement avec une matière d'enrobage entérique et/ou un lubrifiant et/ou un plastifiant, ou analogues.

6. Procédé selon l'une quelconque des revendications 1 à 5, caractérisé en ce que l'enrobage au moyen de la matière d'enrobage pelliculaire est réalisé selon une quantité de 35 à 45 % en poids par rapport aux granules.

7. Composition, à libération prolongée, d'un ingrédient ou d'ingrédients actifs et solubles dans l'eau, comprenant :
   a. un noyau inerte,
   b. une couche d'enrobage pulvérulent, contenant l'ingrédient ou les ingrédients actifs et solubles dans l'eau,
   c. une couche d'enrobage pulvérulent, constituée d'une matière hydrofuge, et
   d. une couche d'enrobage pelliculaire, composée d'une matière d'enrobage à pH sensiblement neutre et insoluble dans l'eau.

## Ansprüche

1. Formulierung mit verzögerter Freigabe eines wasserlöslichen aktiven Bestandteils oder von Bestandteilen, umfassend:
   a) einen inerten Kern;
   b) eine Pulverüberzugsschicht, die den wasserlöslichen aktiven Bestandteil oder die Bestandteile enthält;
   c) eine Pulverüberzugsschicht aus einem wasserabstoßenden Material; und
   d) eine Filmüberzugsschicht, zusammengesetzt aus einem im wesentlichen pH-unabhängigen und wasserunlöslichen Filmüberzugsmaterial.

2. Formulierung mit verzögerter Freigabe nach Anspruch 1, wobei das wasserabstoßende Material, die Schicht (c) und die Schicht aus dem wasserunlöslichen Filmüberzugsmaterial (d) jeweils in Mengen von 10 bis 80 Gew.-% bzw. 20 und 60 Gew.-%, bezogen auf das Gesamtgewicht der Schichten (a) und (b) vorhanden sind.

3. Verfahren zur Herstellung einer Formulierung mit verzögerter Freigabe eines wasserlöslichen aktiven Bestandteils oder von Bestandteilen, umfassend:
das Überziehen eines Kernmaterials mit einem Material, das den wasserlöslichen aktiven Bestandteil oder die Bestandteile in Anwesenheit eines wasserunlöslichen oder sehr schwach löslichen Bindemittels enthält, so daß Granalien hergestellt werden, das Überziehen der Granalien mit einem wasserabstoßenden Material in einer Menge von 10 bis 80 Gew.-%, bezogen auf die Granalien, und das sich daran anschließende Überziehen mit einem im wesentlichen pH-unabhängigen und wasserunlöslichen Filmüberzugsmaterial in einer Menge von 20 bis 60 Gew.-%, bezogen auf die Granalien.

4. Verfahren nach Anspruch 3, wobei sowohl das Material, das den aktiven Bestandteil (Bestandteile) enthält, als auch das wasserabstoßende Material Pulver sind.

5. Verfahren nach Anspruch 3 oder Anspruch 4, wobei eine Trocknungsstufe sich nach dem Überziehen mit dem wasserabstoßenden Material und nach dem Überziehen mit dem Filmüberzugsmaterial anschließt.

6. Verfahren nach einem der Ansprüche 3 bis 5, wobei das Überziehen mit dem Filmüberzugsmaterial zusammen mit einem enterischen Überzugsmaterial und/oder Gleitmittel und/oder Plastifiziermittel oder dergleichen durchgeführt wird.

7. Verfahren nach einem der Ansprüche 3 bis 6, wobei das Überziehen mit dem Filmüberzugsmaterial in einer Menge von 35 bis 45 Gew.-%, bezogen auf die Granalien durchgeführt wird.

Patentansprüche für folgende Vertragsstaaten : ES, GR

1. Verfahren zur Herstellung einer Formulierung mit verzögerter Freigabe eines wasserlöslichen aktiven Bestandteils oder von Bestandteilen, umfassend:
   a) einen inerten Kern,
   b) eine Pulverüberzugsschicht, die den wasserlöslichen aktiven Bestandteil oder Bestandteile enthält;
   c) eine Pulverüberzugsschicht aus einem wasserabstoßenden Material; und
   d) eine Filmüberzugsschicht, die aus einem im wesentlichen pH-unabhängigen und wasserunlöslichen Filmüberzugsmaterial zusammengesetzt ist, umfassend: das Überziehen eines Kernmaterials mit einem Material, das den wasserlöslichen aktiven Bestandteil oder die Bestandteile in Anwesenheit eines wasserunlöslichen oder sehr schwach löslichen Bindemittels enthält, wobei Granalien hergestellt werden, das Überziehen der Granalien mit einem wasserabstoßenden Material und das anschließende Überziehen mit einem im wesentlichen pH-unabhängigen und wasserunlöslichen Filmüberzugsmaterial.

2. Verfahren nach Anspruch 1, wobei das wasserabstoßende Material und das wasserunlösliche Filmüberzugsmaterial in Mengen von entsprechend 10 bis 80 Gew.-% und 20 bis 60 Gew.-%, bezogen auf die Granalien, aufgetragen werden.

3. Verfahren nach Anspruch 1 oder 2, wobei sowohl das Material, das den aktiven Bestandteil (Bestandteile) enthält, wie auch das wasserabstoßende Material Pulver sind.

4. Verfahren nach einem der Ansprüche 1 bis 3, wobei eine Trocknungsstufe nach dem Überziehen mit dem wasserabstoßenden Material und nach dem Überziehen mit dem Filmüberzugsmaterial durchgeführt wird.

5. Verfahren nach einem der Ansprüche 1 bis 4, wobei das Überziehen mit dem Filmüberzugsmaterial zusammen mit einem enterischen Überzugsmaterial und/oder Gleitmittel und/oder Plastifiziermittel oder dergleichen durchgeführt wird.

13

6. Verfahren nach einem der Ansprüche 1 bis 5, wobei das Überziehen mit dem Filmüberzugsmaterial in einer Menge von 35 bis 45 Gew.-%, bezogen auf die Granalien durchgeführt wird.

7. Formulierung mit verzögerter Freigabe eines wasserlöslichen aktiven Bestandteils oder von Bestandteilen, umfassend:

a) einen inerten Kern;

b) eine Pulverüberzugsschicht, die den wasserlöslichen aktiven Bestandteil oder Bestandteile enthält;

c) eine Pulverüberzugsschicht eines wasserabstoßenden Materials; und

d) eine Filmüberzugsschicht, zusammengesetzt aus einem im wesentlichen pH-unabhängigen und wasserunlöslichen Filmüberzugsmaterial.

FIGURE 1. — DISSOLUTION (%) / PPA vs TIME

FIGURE 2. — DISSOLUTION (%) / d-CHLORPHENIRAMINE MALEATE vs TIME

FIGURE 3. — DISSOLUTION (%) / BELLADONNA TOTAL ALKALOIDS vs TIME

FIGURE 4.

FIGURE 5.